# EUROPÄISCHE PATENTANMELDUNG

(11) **EP 0 152 591 A2**
(43) Veröffentlichungstag der Anmeldung: **28.08.1985**
(21) Anmeldenummer: 84115363.8
(22) Anmeldetag: 13.12.1984
(51) Int. Cl.: C07C 161/02, A01N 47/48

(54) **2-Thiocyanato-benzamide**

(30) Priorität: 24.12.1983 DE 3347073
(71) Anmelder: BAYER AG, 51368 Leverkusen (DE)
(72) Erfinder: Schmitt, Hans-Georg, Dr., D-5090 Leverkusen 1 (DE); Wedemeyer, Karlfried, Dr., D-5000 Köln 80 (DE); Brandes, Wilhelm, Dr., D-5653 Leichlingen (DE); Reinecke, Paul, Dr., D-5090 Leverkusen 3 (DE)

(57) **Zusammenfassung**

2-Thiocyanato-benzamide der allgemeinen Formel (I) 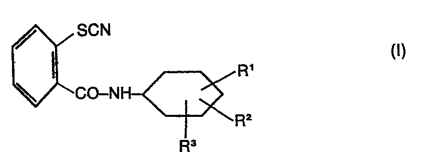 In welcher
R¹, R² und R³ gleich oder verschieden sind und für einen aliphatischen Rest, einen gegebenenfalls ein- oder mehrfach, gleich oder verschieden substituierten Arylalkyl- rest oder einen gegebenenfalls ein- oder mehrfach, gleich oder verschieden substituierten Cycloalkyl-alkyl-Rest stehen oder
R¹ und R² auch unabhängig voneinander für Wasserstoff stehen;
sowie ihre Verwendung als Pflanzenschutzmittel.

Die neuen 2-Thiocyanato-benzamide können hergestellt werden, wenn man z.B, geeignete 2-Mercapto- benzoesäureamide mit einem geeigneten Halogencyan umsetzt.

## Beschreibung

Die vorliegende Erfindung betrifft neue 2-Thiocyanato-benzamide, ein Verfahren zu ihrer Herstellung sowie ihre Verwendung als Pflanzenschutzmittel.

Es sind bereits eine Reihe von 2-Thiocyanato-benzamiden, wie z.B. das 2-Thiocyanato-N-cyclohexyl-benzamid, bekannt geworden, ebenso deren Verwendung z.B. zum Schutz von wasserhaltigen Medien wie Kühlwässern, Anstrichmitteln, ölemulsionen vor mikrobiellem Befall (vgl. EP 0 018 100). Uber eine Verwendung zur Bekämpfung von durch Pilzen verursachten Pflanzenkrankheiten ist nichts bekannt.

Es wurden neue 2-Thiocyanato-benzamide der Formel (I) 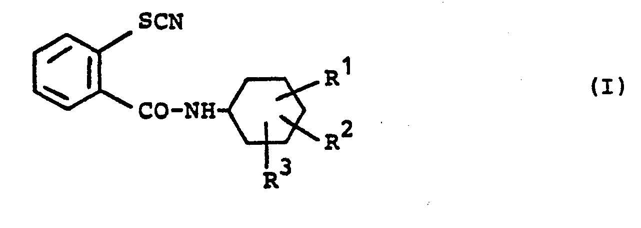 gefunden, in welcher
_{R}¹,_{R}²,_{R}³ gleich oder verschieden sind und für einen aliphatischen Rest, einen gegebenenfalls ein-oder mehrfach, gleich oder verschieden substituierten Arylalkylrest oder einen gegebenenfalls ein- oder mehrfach, gleich oder verschieden substituierten Cycloalkyl-alkylrest stehen oder
R¹ und R² unabhängig voneinander für Wasserstoff stehen. Weiterhin wurde gefunden, daß man die 2-Thiocyanato-benzamide der Formel (I) 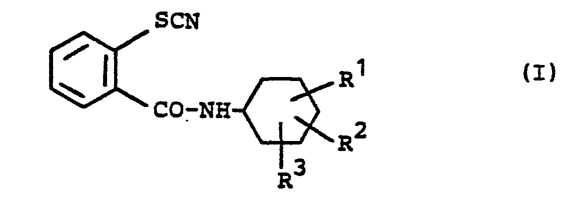 in welcher
R¹,R²,R³ gleich oder verschieden sind und für einen aliphatischen Rest, einen gegebenenfalls ein-oder mehrfach, gleich oder verschieden substituierten Arylalkyl-Rest oder einen gegebenenfalls ein- oder mehrfach, gleich oder verschieden substituierten Cycloalkyl-alkyl-Rest stehen, oder
R¹ und R² unabhängig voneinander auch für Wasserstoff stehen können, erhält,
wenn man 2-Mercapto-benzoesäureamide der Formel (II) in welcher 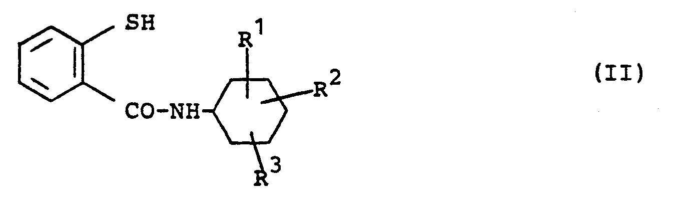
R¹, _{R}² und _{R}³ die oben angegebene Bedeutung haben, mit einer Verbindung der Formel (III) 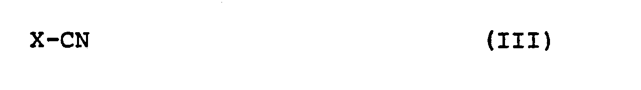 in welcher
X für Halogen steht, bevorzugt für Chlor und Brom,
gegebenenfalls in Gegenwart einer Base oder eines sauren oder basischen Katalysators und gegebenenfalls in Gegenwart eines Verdünnungsmittels bei Temperaturen von -40°C bis +60°C umsetzt.

Die erfindungsgemäßen 2-Thiocyanato-benzamide der Formel (I) weisen starke fungizide Eigenschaften auf. Uberraschenderweise zeigen dabei die erfindungsgemäßen Verbindungen eine erheblich höhere Wirkung als das aus dem Stand der Technik bekannte und strukturell naheliegende 2-Thiocyanato-N-cyclohexyl-benzamid. Die neuen Verbindungen stellen somit eine Bereicherung der Technik dar.

Von den neuen 2-Thiocyanato-benzamiden der Formel (I) sind bevorzugt diejenigen, bei denen
_{R}¹,_{R}² und R³ gleich oder verschieden sind und für einen geradkettigen, verzweigten oder cyclischen Alkylrest mit bis zu 8 Kohlenstoffatomen, einen gegebenenfalls ein- bis fünffach durch Alkyl mit 1 bis 4 Kohlenstoffatomen substituierten Phenylalkylrest mit 1 bis 4 Kohlenstoffatomen, insbesondere 1 oder 2 Kohlenstoffatomen, im Alkylrest oder einen Cycloalkyl-alkylrest mit 5 bis 8 Kohlenstoffatomen im Cycloalkylteil und 1 bis 4, insbesondere 1 oder 2 Kohlenstoffatomen, im Alkylteil stehen oder
_{R}¹ und R 2 unabhängig voneinander für Wasserstoff stehen.

Besonders bevorzugt sind 2-Thiocyanato-benzamide der Formel (I), in welcher
R¹,R² und _{R}³ gleich oder verschieden sind und für geradkettiges oder verzweigtes Alkyl mit 1 bis 4 Kohlenstoffatomen, für Cycloalkyl mit 5 bis 8 Kohlenstoffatomen, für gegebenenfalls einfach bis dreifach, gleich oder verschieden durch Methyl und Ethyl substituiertes Phenylmethyl, Phenylethyl, Cyclohexylmethyl oder Cyclohexylethyl stehen oder
_{R}¹ und _{R}² unabhängig voneinander für Wasserstoff stehen.

Ganz besonders bevorzugt sind 2-Thiocyanato-benzamide der Formel (I), in welcher
R¹,R²,R³ gleich oder verschieden sind und für Methyl, Ethyl, n- und iso-Propyl, Cyclohexyl, für gegebenenfalls ein- oder zweifach durch Methyl substituiertes Benzyl oder Cyclohexylmethyl stehen oder
_{R}¹ und _{R}² unabhängig voneinander für Wasserstoff stehen.

Hinsichtlich der Substituenten im Cyclohexylrest kann es sich dabei um die reinen Stereoisomeren als auch um Gemische von verschiedenen Stereoisomeren handeln.

Neben den zu den Herstellungsbeispielen genannten Verbindungen der Formel (I) seien noch im einzelnen die folgenden Verbindungen genannt:
2-Thiocyanato-benzoesäure-N-(2-methyl-cyclohexyl)-amid,
2-Thiocyanato-benzoesäure-N-(3,5-dimethyl-cyclohexyl)-amid,
2-Thiocyanato-benzoesäure-N-(2-ethyl-cyclohexyl)-amid,
2-Thiocyanato-benzoesäure-N-(3-ethyl-cyclohexyl)-amid,
2-Thiocyanato-benzoesäure-N-(4-ethyl-cyclohexyl)-amid,
2-Thiocyanato-benzoesäure-N-(2,6-dimethyl-cyclohexyl)-amid,
)2-Thiocyanato-benzoesäure-N-(2,6,6-trimethyl-cyclohexyl)-amid,
2-Thiocyanato-benzoesäure-N-(3-isopropyl-5-methyl-cyclohexyl)-amid,
2-Thiocyanato-benzoesäure-N-(3-isopropyl-6-methyl-cyclohexyl)-amid,
2-Thiocyanato-benzoesäure-N-(2,4,6-trimethyl-cyclohexyl)-amid,
2-Thiocyanato-benzoesäure-N-(4-propyl-cyclohexyl)-amid,
2-Thiocyanato-benzoesäure-N-(3-isopropyl-cyclohexyl)-amid,
2-Thiocyanato-benzoesäure-N-(4-cyclohexylmethyl-cyclohexyl)-amid,
2-Thiocyanato-benzoesäure-N-(3-cyclohexyl-cyclohexyl)-amid,
2-Thiocyanato-benzoesäure-N-(4-cyclohexyl-cyclohexyl)-amid,
2-Thiocyanato-benzoesäure-N-(2-cyclohexyl-cyclohexyl)-amid und
2-Thiocyanato-benzoesäure-N-(2-isopropyl-5-methyl-cyclohexyl)-amid.

Die Herstellung der erfindungsgemäßen Verbindungen der Formel (I) kann durch folgendes Formelschema veranschaulicht werden, wenn man beispielsweise 2-Mercapto- benzoesäure-N-(2-ethyl-cyclohexyl)-amid und Chlorcyan als Ausgangsstoffe verwendet:

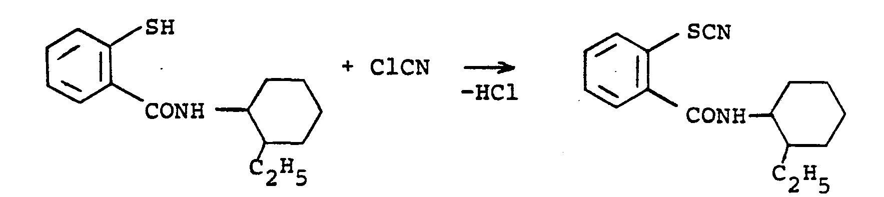

Die als Ausgangsverbindungen zu verwendenden 2-Mercapto- benzoesäureamide sind durch die Formel (II) allgemein definiert. Die Verbindungen sind bekannt und/oder können nach bekannten Verfahren hergestellt werden.

So kann man sie zum Beispiel auf folgendem Weg synthetisieren: Ausgehend von Diphenyldisulfid-2,2'-dicarbonsäure und Thionylchlorid wird das Disäurechlorid hergestellt, das mit einem primären Amin zum Dithio-bisbenzoesäureamid reagiert. Durch Reduktion z.B. mit Zinkpulver lassen sich daraus die 2-Mercaptobenzoesäureamide herstellen.

Die ebenfalls als Ausgangsprodukte einzusetzenden Halogencyane der Formel (II) sind allgemein bekannte Verbindungen.

Die Umsetzung der 2-Mercaptobenzamide mit dem Halogencyan wird vorzugsweise in Gegenwart eines Verdünnungsmittels durchgeführt. Als Verdünnungsmittel kommen praktisch alle inerten organischen Lösemittel in Frage. Hierzu gehören insbesondere Alkohole wie z.B. Methanol und Ethanol; Nitrile wie Acetonitril; Aromaten wie Toluol; Ether wie Tetrahydrofuran, Glykoldimethylether und Dioxan; Amide ::i= Dimethylformamid und N-Methylpyrrolidinon. Es können auch Gemische von Verdünnungsmitteln eingesetzt werden, so z.B. zweiphasige Gemische aus einem Aromaten und Wasser.

Die Reaktionstemperatur kann in einem weiten Bereich variiert werden. Normalerweise arbeitet man zwischen -40°C und +60°_{C}, bevorzugt zwischen -20°C und +40°_{C}.

Die Reaktion kann gegebenenfalls in Gegenwart einer Base durchgeführt werden. Geeignet sind Alkali- und Erdalkalihydroxide wie Natrium- und Kaliumhydroxid, Magnesiumhydroxid, Bariumhydroxid, Alkalicarbonate wie Natriumcarbonat und Kaliumcarbonat; tertiäre Amine wie Triethylamin, Dimethyl-benzylamin und 1,4-Diaza-bicyclo-(2.2.2)octan. Der Anteil der Base im Verhältnis zum Mercapto-benzamid kann 0,1 bis 100 Mol-% betragen. Es ist jedoch auch möglich, die Reaktion in Gegenwart saurer Katalysaturen durchzuführen; als solche kommen in Frage: Mineralsäuren wie Salzsäure und Schwefelsäure; Carbonsäuren wie Essigsäure.

Der Anteil des sauren Katalysators im Verhältnis zum Mercaptobenzamid kann 0,1 bis 20 Mol-% betragen.

Das Mercapto-benzamid und das Halogencyan werden im allgemeinen in äquimolaren Mengen eingesetzt. Es kann jedoch vor allem bei Verwendung von Chlorcyan vorteilhaft sein, dieses im Überschuß einzusetzen. Der Überschuß kann dabei bis zu 20 Mol im Verhältnis zum Mercapto- benzamid betragen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

So können z.B. fungizide Mittel im Pflanzenschutz eingesetzt werden zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Botrytis-Arten, wie beispielsweise Botrytis cinerea;
Plasmopara-Arten, wie beispielsweise Plasmopara viticola, Uromyces-Arten, wie beispielsweise Uromyces appendiculatus;
Sphaerotheca-Arten, wie beispielsweise Sphaerotheca fuliginea;
Venturia-Arten, wie beispielsweise Venturia inaequalis;
Podosphaera-Arten, wie beispielsweise Podosphaera leucotricha;
Phytophthora-Arten, wie beispielsweise Phytophthora infestans;
Erysiphe-Arten, wie beispielsweise Erysiphe graminis;
Puccinia-Arten, wie beispielsweise Puccinia recondita;
Fusarium-Arten, wie beispielsweise Fusarium culmorum;
Ustilago-Arten, wie beispielsweise Ustilago nuda oder
Septoria-Arten, wie beispielsweise Septoria nodorum;
Tilletia-Arten, wie beispielsweise Tilletia caries;
^{p}yricularia-Arten, wie beispielsweise Pyricularia oryzae;
Pellicularia-Arten, wie beispielsweise Pellicularia sasakii;
Pyrenophora-Arten, wie beispielsweise Pyrenophora teres (Konidienform: Drechslera, Syn: Helminthosporium); Leptosphaeria-Arten, wie beispielsweise Leptosphaeria nodorum, Cochliobolus-Arten, wie beispielsweise Cochliobolus sativus (Konidienform: Drechslera, Syn: Helminthospcrium); und
Cercospora-Arten, wie beispielsweise Cercospora canescens.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungzmittel verwendet werden. Als flüssige Lösungsmittel kommen im wesentlichen in Frage: Aromaten, wie Xyicl, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarvlpolyglycol-ether, Alkylsulfonatc, Alkylsulfate. Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden. Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Einige der Verbindungen sind bei entsprechender Anwendung auch wirksam gegen Reiskrankheiten (Pyricularia oryzae) und zeigen eine gute Wirkung im in-vitro Plattentest, die gute Wirkung gegen die verschiedensten Getreidekrankheiten seien erwähnt.

Das erfindungsgemäße Verfahren wird durch die folgenden Herstellungsbeispiele erläutert, aber nicht darauf beschränkt.

### Herstellungsbeispiele

### Beispiel 1

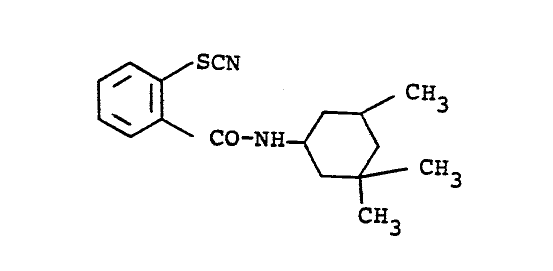

49,9 g (0,18 Mol) 2-Mercaptobenzoesäure-N-(3,5,5-trimethyl cyclohexyl)-amid werden in 200 ml Tetrahydrofuran vorgelegt. Die Lösung wird auf -15°C gekühlt und 33,3 g (0,54 Mol) Chlorcyan in 50 ml Tetrahydrofuran gelöst werden innerhalb von 30 Minuten so zugetropft, daß die Innentemperatur -5°C nicht übersteigt. Nach Beendigung des Zutropfens wird innerhalb 1 Stunde auf Raumtemperatur kommen gelassen, dann wird weitere 2 Stunden bei dieser Temperatur weitergerührt. überschüssiges Chlorcyan und ein Teil des Lösungsmittels werden unter vermindertem Druck abdestilliert. Die Lösung wird unter starkem Rühren in Wasser eingetragen, der abgeschiedene Feststoff abgesaugt und im Exsiccator getrocknet. Man erhält 47 g (86,5 % der Theorie) an 2-Thiocyanato-benzoesäure-N-(3,5,5-trimethylcyclohexyl)-amid als farbloses Pulver mit einem Schmelzpunkt von 117°C.

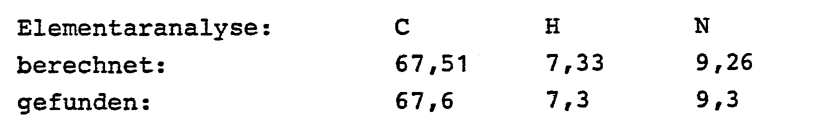

Ebenso wurden erhalten die Verbindungen der Formel (I)

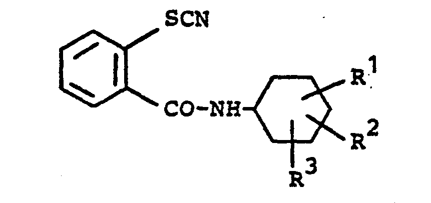

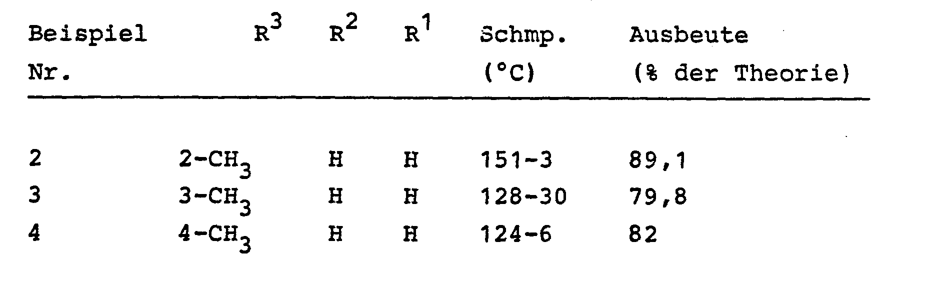

### Anwendungsbeispiele

In den folgenden Anwendungsbeispielen werden die nachstehend aufgeführten Verbindungen als Vergleichssubstanzen eingesetzt: 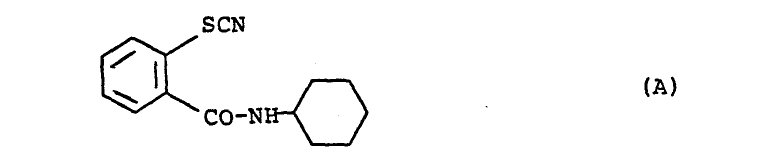 2-Thiocyanato-benzoesäure-N-cyclohexyl-amid 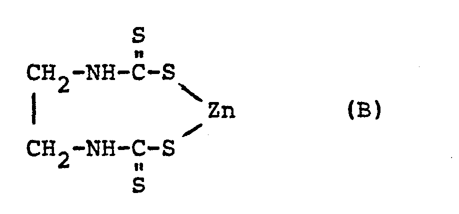 Zinkethylen-1,2-bis-dithiocarbamat

### Beispiel A

Venturia-Test (Apfel) / protektiv

Lösungsmittel: 4,7 Gewichtsteile Aceton Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß der Herstellungsbeispiele: 1 und 2

### Beispiel B

Phytophthora-Test (Tomate) / kurativ

Lösungsmittel: 4,7 Gewichtsteile Aceton Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Sporensuspension von Phytophthora infestans inokuliert. Die Pflanzen verbleiben 7 Stunden bei 20°C und 100 % relativer Luftfeuchtigkeit in einer Inkubationskabine. Nach einer kurzen Abtrocknungszeit werden die Pflanzen mit der Wirkstoffzubereitung tropfnaß gespritzt.

Die Pflanzen werden in einer Inkubationskabine mit 100 % relativer Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß der Herstellungsbeispiele: 1 und 2

### Beispiel C

Erysiphe-Test (Gerste) / protektiv

Lösungsmittel: 100 Gewichtsteile Dimethylformamid Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele:
1 und 4

### Beispiel D

Leptosphaeria nodorum-Test (Weizen / protektiv

Lösungsmittel: 100 Gewichtsteile Dimethylformamid Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100% relativer Luftfeuchtigkeit in einer Inkubationskabine.

Die Pflanzen werden in einem Gewächshaus bei einer Temperatur von ca. 15°C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 1 + 4

## Patentansprüche

1. 2-Thiocyanato-benzamide der allgemeinen Formel (I) in welcher 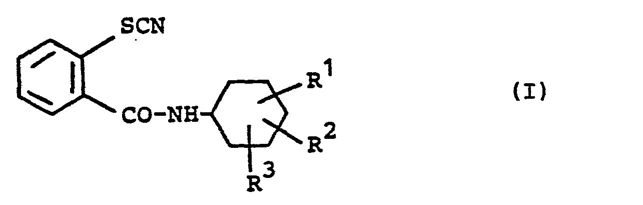
_{R}¹,_{R}²,_{R}³ gleich oder verschieden sind und für einen aliphatischen Rest, einen gegebenenfalls ein- oder mehrfach, gleich oder verschieden substituierten Arylalkylrest oder einen gegebenenfalls ein- oder mehrfach, gleich oder verschieden substituierten Cycloalkyl-alkyl-Rest stehen, oder
R¹ und R² unabhängig voneinander für Wasserstoff stehen.

2. 2-Thiocyanato-benzamide der Formel (I) gemäß Anspruch 1, in welcher
R¹,R² und _{R}³ gleich oder verschieden sind und für einen geradkettigen, verzweigten oder cyclischen Alkylrest mit bis zu 8 Kohlenstoffatomen, einen gegebenenfalls ein- bis fünffach durch Alkyl mit 1 bis 4 Kohlenstoffatomen substituierten Phenylalkylrest mit 1 bis 4 Kohlenstoffatomen im Alkylrest oder einen Cycloalkyl-alkylrest mit 5 bis 8 Kohlenstoffatomen im Cycloalkylteil und 1 bis 4 Kohlenstoffatomen im Alkylteil stehen oder
_{R}¹ und _{R}² unabhängig voneinander für Wasserstoff stehen.

3. 2-Thiocyanato-benzamide der Formel (I) gemäß Anspruch 1, in welcher
_{R}¹, _{R}² und _{R}³ gleich oder verschieden sind und für geradkettiges oder verzweigtes Alkyl mit 1 bis 4 Kohlenstoffatomen, für Cycloalkyl mit 5 bis 8 Kohlenstoffatomen, für gegebenenfalls ein- bis dreifach, gleich oder verschieden durch Methyl und Ethyl substituiertes Phenylmethyl, Phenylethyl, Cyclohexylmethyl oder Cyclohexylethyl stehen oder
_{R}¹ und R² unabhängig voneinander für Wasserstoff stehen.

4. 2-Thiocyanato-benzamide der Formel (I) gemäß Anspruch 1, in welcher
_{R}¹, _{R}², R3 gleich oder verschieden sind und für Methyl, Ethyl, n- und iso-Propyl, Cyclohexyl, für gegebenenfalls ein-oder zweifach durch Methyl substituiertes Benzyl oder Cyclohexylmethyl stehen oder
R¹ und R² unabhängig voneinander für Wasserstoff stehen.

5. Verfahren zur Herstellung von 2-Thiocyanato-benzamiden der Formel (I) in welcher 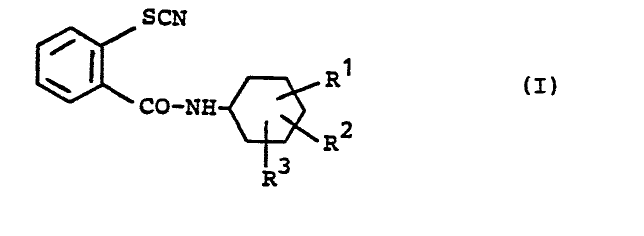
_{R}¹,_{R}²,_{R}³ gleich oder verschieden sind und für einen aliphatischen Rest, einen gegebenenfalls ein- oder mehrfach, gleich oder verschieden substituierten Arylalkyl-Rest oder einen gegebenenfalls ein- oder mehrfach, gleich oder verschieden substituierten Cycloalkyl-alkyl-Rest stehen, oder
_{R}¹ und _{R}² unabhängig voneinander für Wasserstoff stehen, dadurch gekennzeichnet, daß man 2-Mercapto-benzoesäureamide der Formel (II) in welcher 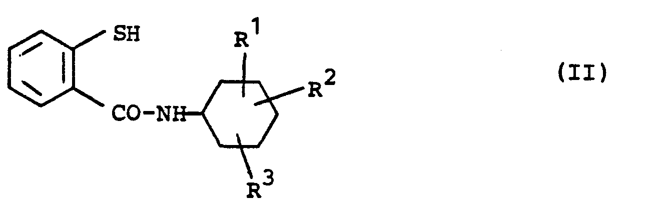
R¹, R² und _{R}³ die oben angegebene Bedeutung haben, mit einer Verbindung der Formel (III) 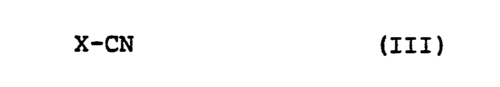 in welcher
X für Halogen steht,
gegebenenfalls in Gegenwart einer Base oder eines sauren oder basischen Katalysators, gegebenenfalls in Gegenwart eines Verdünnungsmittels bei Temperaturen von -40°C bis +60°C umsetzt.

6. Pflanzenschutzmittel, gekennzeichnet durch einen Gehalt an mindestens einem 2-Thiocyanato-benzamid der Formel (I) gemäß den Ansprüchen 1 und 5.

7. Verwendung von 2-Thiocyanato-benzamiden der Formel (I) gemäß den Ansprüchen 1 und 5 zur Bekämpfung von Pilzen.

8. Verfahren zur Bekämpfung von Pilzen, dadurch gekennzeichnet, daß man 2-Thiocyanato-benzamide der Formel (I) gemäß den Ansprüchen 1 und 5 auf Pilze und/oder ihren Lebensraum einwirken läßt.

9. Verfahren zur Herstellung von Pflanzenschutzmitteln, dadurch gekennzeichnet, daß man 2-Thiocyanato-benzamide der Formel (I) gemäß den Ansprüchen 1 und 5 mit Streckmitteln und/oder oberflächenaktiven Mitteln vermischt.
